# EUROPÄISCHE PATENTANMELDUNG

(11) **EP 2 422 826 A2**
(43) Veröffentlichungstag der Anmeldung: **29.02.2012**
(21) Anmeldenummer: 11176428.8
(22) Anmeldetag: 03.08.2011
(51) Int. Cl.: A61L 31/02, A61L 31/08

(54) **Implantat und Verfahren zur Herstellung desselben**

(30) Priorität: 27.08.2010 US 377474 P
(71) Anmelder: Biotronik AG, 8180 Bülach (CH)
(72) Erfinder: Fircho, Horst, 18184 Kösterbeck (DE); Bayer, Ullrich, 18211 Admannshagen-Bargeshagen (DE); Endres, Frank, 38678 Clausthal-Zellerfeld (DE)
(74) Vertreter: Lindner-Vogt, Karin L.

(57) **Zusammenfassung**

Die vorliegende Erfindung schlägt ein Verfahren zur Herstellung eines Implantats, insbesondere einer intraluminalen Endoprothese, mit einem Körper vor, wobei der Körper Eisen oder eine Eisenlegierung aufweist, umfassend die folgenden Schritte:

a) Bereitstellen des Implantatkörpers (1),
b) Aufbringen einer metallischen Beschichtung mit dem Hauptbestandteil mindestens eines Elements der Gruppe enthaltend Tantal, Niob, Zirkon, Aluminium, Magnesium, Vanadium, Molybdän, Hafnium und Wolfram auf mindestens einen Teil der Körperoberfläche,
c) plasmachemische Behandlung des mit der Beschichtung versehenen Teils der Körperoberfläche in einer wässrigen Lösung zur Herstellung einer plasmachemisch erzeugten Schicht (3, 5, 7) mittels Anlegen einer das Plasma generierenden Mischspannung an den Körper des Implantats, wobei die Mischspannung eine ausreichende Energie dafür aufweist, dass die metallische Beschichtung zeitweise und abschnittsweise derart bis zu dem darunter liegenden Implantatkörper (1) ionisiert wird, dass die plasmachemisch erzeugte Schicht (3, 5, 7) zumindest teilweise bis zum Implantatkörper durchgehende Poren (5) aufweist.

Die Erfindung betrifft ferner ein Implantat erhältlich durch ein derartiges Verfahren.

## Beschreibung

Die Erfindung betrifft ein Verfahren zur Herstellung eines Implantats, insbesondere einer intraluminalen Endoprothese, mit einem Körper, wobei der Körper Eisen oder eine Eisenlegierung enthält, sowie ein Implantat erhältlich oder erhalten durch ein derartiges Verfahren.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents, Befestigungselemente für Knochen, beispielsweise Schrauben, Platten oder Nägel, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heutzutage werden als Implantate besonders häufig Stents eingesetzt, die zur Behandlung von Stenosen (Gefäßverengungen) dienen. Sie weisen einen Körper in der Form eines rohrförmigen oder hohlzylinderförmigen Grundgitters auf, das an beiden Längsenden offen ist. Das rohrförmige Grundgitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können.

Ein Ansatzpunkt zur Lösung dieses Problems besteht darin, den Stent bzw. andere Implantate aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Körper biodegradierbarer Implantate geeignete Werkstoffe können beispielsweise Polymere oder Metalle enthalten. Der Körper kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Die vorliegende Erfindung bezieht sich auf Implantate mit einem metallischen biodegradierbaren Werkstoff basierend auf Eisen oder Eisenbasis-Legierungen (im Folgenden kurz: Eisenlegierung).

Es sind bereits Stents bekannt, die Beschichtungen mit verschiedenen Funktionen aufweisen. Derartige Beschichtungen dienen beispielsweise der Freisetzung von Medikamenten, der Anordnung eines Röntgenmarkers oder dem Schutz der darunter liegenden Strukturen. Bei der Realisierung biodegradierbarer Implantate soll die Degradierbarkeit entsprechend der angestrebten Therapie bzw. der Anwendung des jeweiligen Implantats (koronar, intracranial, renal etc.) gesteuert werden. Für viele therapeutische Anwendungen ist beispielsweise ein wichtiger Zielkorridor, dass das Implantat in einem Zeitraum von vier Wochen bis sechs Monaten seine Integrität verliert. Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, dem Aufhalten des Gefäßes, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben aufweist.

Implantate mit einer Eisenlegierung, insbesondere eisenhaltige Stents, sind besonders kostengünstig und einfach herstellbar. Beispielsweise für die Behandlung von Stenosen verlieren diese Implantate allerdings ihre mechanische Integrität bzw. Stützwirkung erst nach einem vergleichsweise langen Zeitraum, d. h. erst nach einer Verweildauer in dem behandelten Organismus von ca. 2 Jahren. Dies bedeutet, dass sich der Kollapsdruck bei eisenhaltigen Implantaten für die gewünschten Anwendungen über der Zeit zu langsam verringert.

Im Stand der Technik sind bereits unterschiedliche Mechanismen der Degradationskontrolle von Implantaten beschrieben. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material beruhen. Diese führen dazu, dass die Degradationszeit verlängert wird. So wird der Degradationsschutz durch verschieden zusammengesetzte Schutzschichten und durch definierte geometrische Abstände (Diffusionsbarrieren) zwischen dem Korrosionsmedium und dem Magnesiumgrundmaterial abgesichert. Andere Lösungen basieren darauf, Legierungsbestandteile des biodegradierbaren Materials des Implantatkörpers gezielt zu verändern. Mit den zuvor genannten Lösungen gelingt es jedoch meist nicht, die durch den Degradationsprozess eintretende Auflösung und die daraus resultierenden Stegbrüche in das geforderte Zeitfenster zu legen. Die Folge ist insbesondere bei Implantaten mit einem Körper enthaltend eine Eisenlegierung eine zu spät einsetzende bzw. eine zu große Variabilität der Degradation des Implantats.

Ein weiteres Problem im Zusammenhang mit Beschichtungen ergibt sich auch daraus, dass Stents oder andere Implantate üblicherweise zwei Zustände annehmen, nämlich einen komprimierten Zustand mit einem kleinen Durchmesser und einen expandierten Zustand mit einem größeren Durchmesser. Im komprimierten Zustand kann das Implantat mittels eines Katheters in das zu stützende Gefäß eingeführt und an der zu behandelnden Stelle positioniert werden. Am Ort der Behandlung wird das Implantat dann beispielsweise mittels eines Ballonkatheters dilatiert. Auf Grund dieser Durchmesseränderung ist der Körper des Implantats hierbei einer starken mechanischen Belastung ausgesetzt. Weitere mechanische Belastungen des Implantats können während der Herstellung oder bei der Bewegung des Implantats im oder mit dem Gefäß, in das das Implantat eingesetzt ist, auftreten. Bei den oben genannten Beschichtungen ergibt sich somit der Nachteil, dass eine Beschichtung während der Verformung des Implantats reißt (z. B. Bildung von Mikrorissen) oder auch teilweise entfernt wird. Hierdurch kann eine unspezifizierte lokale Degradation verursacht werden. Außerdem sind das Einsetzen und die Geschwindigkeit der Degradation von der Größe und der Verteilung der durch die Verformung entstehenden Mikrorisse abhängig, die als Fehlstellen schlecht kontrollierbar sind. Dies führt zu einer starken Streuung in den Degradationszeiten.

Aus den Druckschriften US 2008/0086195 A1 und WO 2008/045184 A1 ist eine medizinische Vorrichtung wie ein Katheter oder ein Stent bekannt, bei dem eine polymerfreie Beschichtung mittels eines plasmaelektrolytischen Prozesses (plasma electrolytic deposition PED) aufgebracht wird. Die plasmaelektrolytische Beschichtung wird dafür verwendet, zusätzlich Wirkstoffe in die Beschichtung einzubringen, welche ein Medikament oder ein therapeutisches Mittel beinhalten. Die plasmaelektrolytischen Beschichtung umfasst eine plasmaelektrische Oxidation (plasma electrolytic oxidation PEO), eine Mikro-Lichtbogenoxidation (micro-arc oxidation MAO), eine Plasma-Lichtbogenoxidation (plasma-arc oxidation PAO), eine anodische Funkenoxidation (anodic spark oxidation) und eine elektrolytische Plasmasättigung (plasma electrolytic saturation PES). Die plasmaelektrolytische Beschichtung wird mittels Wechselspannung durchgeführt. Die plasmaelektrolytische Behandlung beinhaltet die Anwendung von verschiedenen elektrischen Potentialen zwischen der medizinischen Vorrichtung und einer Gegenelektrode, die eine elektrische Entladung (eine Funken- oder Lichtbogenplasmamikro-Entladung) an oder in der Nähe der Oberfläche der medizinischen Vorrichtung erzeugt, welche keine wesentliche Verlängerung der Degradationszeiten bewirkt. Das in den genannten Druckschriften angegebene Verfahren löst somit nicht das oben angegebene Problem.

Folglich besteht die Aufgabe der vorliegenden Erfindung darin, ein kostengünstiges Verfahren zur Herstellung eines Implantats anzugeben, das eine Degradation der Implantate mit Eisen oder einer Eisenlegierung im gewünschten Zeitfenster bewirkt, insbesondere in einem kürzeren Zeitraum. Dabei soll die Degradation zu einem kontrollierbaren Zeitpunkt stattfinden. Entsprechend besteht die Aufgabe der Erfindung außerdem darin, ein derartiges Implantat zu schaffen.

Die obige Aufgabenstellung wird gelöst durch ein Verfahren umfassend die folgenden Schritte:
a) Bereitstellen des Implantatkörpers,
b) Aufbringen einer metallischen Beschichtung mit dem Hauptbestandteil mindestens eines Elements der Gruppe enthaltend Tantal, Niob, Zirkon, Aluminium, Magnesium, Vanadium, Molybdän, Hafnium und Wolfram auf mindestens einen Teil der Körperoberfläche,
c) plasmachemische Behandlung des mit der Beschichtung versehenen Teils der Körperoberfläche in einer wässrigen Lösung zur Herstellung einer plasmachemisch erzeugten Schicht mittels Anlegen einer das Plasma generierenden Mischspannung an den Körper des Implantats, wobei die Mischspannung eine ausreichende Energie dafür aufweist, dass die metallische Beschichtung zeitweise und abschnittsweise derart bis zu dem darunter liegenden Implantatkörper ionisiert wird, dass die plasmachemisch erzeugte Schicht zumindest teilweise bis zum Implantatkörper durchgehende Poren aufweist.

Bei dem erfindungsgemäßen Verfahren wird zunächst eine metallische Beschichtung mit einem oder mehreren Ventilmetallen wie Tantal, Niob, Zirkon, Aluminium, Magnesium, Vanadium, Molybdän, Hafnium und/oder Wolfram, bevorzugt Tantal, Niob und/oder Zirkon, auf die Körperoberfläche des Implantatkörpers aufgebracht. Anschließend wird eine plasmachemische Behandlung derart durchgeführt, dass Poren in der metallischen Beschichtung erzeugt werden, welche durch die metallische Beschichtung hindurch gehen und bis zur Oberfläche des Implantatkörpers reichen. Dies bedeutet, dass die Poren etwa eine Tiefe aufweisen, welche der Schichtdicke der metallischen Beschichtung nach Schritt b) entspricht. Eine Ionisierung erfolgt daher bis zum Grundmaterial, das Eisen oder eine Eisenlegierung enthält, wobei die Poren in geometrisch regelmäßigen Abständen angeordnet sind.

Hierbei entstehen die Poren jeweils dort, wo die geringste Durchschlagsspannung der Ventilmetallschicht vorliegt und wo folglich der Plasmaprozess gezündet wird. Sind diese Zonen beschichtetet, folgen übergangslos Oberflächenbereiche die - unter anderem auch geometriebedingt - eine höhere Feldliniendichte aufweisen. In Abhängigkeit der konkreten Beschichtungsbedingungen ist jedoch auch eine umgekehrte Reihenfolge im Verlauf der Ausbildung des Plasmas möglich.

Die Regelmäßigkeit der Porenabstände ergibt sich auf Grund der Tatsache, dass Bereiche, in denen das Plasma gezündet wird, immer die jeweilige Schwachstelle hinsichtlich Durchschlagsfestigkeit darstellen. Die unmittelbare Umgebung einer Pore kann auf Grund der vorwiegend oxidisch-/phosphatischen Schichtzusammensetzung als plasmaresistent bezeichnet werden. Erst in einer bestimmten Entfernung vom Rand einer bereits erzeugten Pore (ca. 1 bis 3 µm) nimmt der metallische Anteil des Beschichtungsmaterials wieder zu und es kommt danach zu einer plasmachemischen Oxidation dieser Zonen. Bei einer nach oben begrenzten Spannung (Endspannung) und einer konstanten Stromdichte erfolgt dieses "Abrastern" der Oberfläche bis diese Regelmäßigkeit in der Porenstruktur vorhanden ist. Danach sinkt der Strom schnell ab. Bei Erreichen von ca. 20% der Ausgangsstromdichte kann der Beschichtungsprozess durch manuelles Unterbrechen des Stromflusses abgebrochen werden.

Unter der plasmachemisch erzeugten Schicht wird die aus der metallischen Beschichtung und einem an der Oberfläche des Implantatkörpers angeordneten Volumen des Grundmaterials sowie den Poren durch das plasmachemische Verfahren gebildete Schicht verstanden. Ferner wird unter einer "durch die plasmachemisch erzeugte Schicht durchgehende Pore" verstanden, dass eine dünne, maximal 200 nm dicke, im Querschliff sichtbare Porengrundschicht die Oberfläche des Grundmaterials des Implantatkörpers bedeckt. Diese Porengrundschicht verhindert, dass der Elektrolyt den plasmachemischen Prozess bei Kontakt mit dem Grundmaterial des Implantkörpers nicht stört. Deshalb sind die elektrischen Parameter so zu wählen, dass der Energieeintrag bei der Plasmawirkung an das Grundmaterial nur bis zu einem Abstand von maximal 0,5 µm heranreicht. Dadurch wird bewirkt, dass die Porengründe durch Oxide und Phosphate des Beschichtungsmaterials und einen geringen Anteil an Oxiden und Phosphaten des Grundmaterials des Implantatkörpers, beispielsweise einem geringen Anteil an Oxiden- und Phosphaten einer Eisenbasislegierung, gebildet werden. Rein metallisches Implantatkörpermaterial liegt somit am Porengrund nicht vor. Die Dicke der aus Mischoxiden- und Mischphosphaten aus dem Beschichtungswerkstoff und dem Grundwerkstoff bestehenden Schicht am Porengrund beträgt zwischen ca. 10 nm und 200 nm.

Bei der plasmachemischen Behandlung wird die Körperoberfläche in einem wässrigen Elektrolytsystem (wässrige Lösung) behandelt und die plasmachemischen Effekte entstehen direkt an der Oberfläche der metallischen Beschichtung. Das für Mikrosekunden stabile Plasma an der Oberfläche der metallischen Beschichtung erzeugt Reaktionsprodukte, welche zur Ausbildung der Poren in der metallischen Beschichtung führen. Hierbei werden auch die metallische Beschichtung teilweise und ein Teil der oberflächennahen Schicht der darunter liegenden Oberfläche des Implantatkörpers umgesetzt. Entsprechend weist die durch die plasmachemische Behandlung erzeugte mikroporöse oder perforierte Schicht hinsichtlich ihrer chemischen Zusammensetzung mehrere Phasen auf. An der Oberfläche der plasmachemisch erzeugten Schicht sind Oxide, Phosphate und/oder Hydroxide der Elemente der metallischen Beschichtung, insbesondere des Tantals und/oder des Niobs und/ oder des Zirkons vorhanden. Darunter sind Reste der in Schritt b) aufgebrachten metallischen Beschichtung angeordnet. Im Bereich des Porengrundes sind Eisenverbindungen, Eisenoxide und/oder Eisenphosphate sowie die Oxide und Phosphate der jeweiligen Beschichtungsmetalle zu finden. Darunter liegt das ursprüngliche Material des Implantatkörpers.

Der Körper des Implantats umfasst mindestens einen Teil des Implantats, vorzugsweise den Hauptteil des Implantats, welcher die mechanische Integrität des Implantats bewirkt. Die vorliegende Erfindung bezieht sich dabei insbesondere auf Implantate, deren Material des Körpers Eisen als Hauptbestandteil enthält und vorzugsweise biodegradierbar ist.

Ein ganz wesentlicher Vorteil des erfindungsgemäßen Verfahrens gegenüber dem Stand der Technik besteht darin, dass durch die hohe Energie der das Plasma generierenden Mischspannung (auch Impulsspannung genannt) sowohl die metallische Beschichtung als auch ein darunter liegender Bereich des Implantatkörpers zeitweise und abschnittsweise ionisiert werden. Hierbei steigt die Spannung von anfänglich 0 V über einen Zeitraum von ca. 3 µs auf eine Peakspannung an und fällt dann parabolisch innerhalb der darauf folgenden 2 µs ab. Die Peakspannung wird von Puls zu Puls bis auf einen Maximalwert erhöht, der bei etwa 500 V liegt. Durch die Trägheit des Systems (Elektrolyt, Elektroden, mechanische Trägheit der Ladungsträger im Elektrolyt) geht die Spannung nach Durchlaufen der Peakspannung jedoch nicht mehr auf Null zurück sondern lediglich auf einen Minimalwert. Danach steigt die Spannung wieder bis zur nächsten Peakspannung an usw.. Durch das so generierte Plasma wird die metallische Beschichtung abschnittsweise ionisiert, so dass Poren entstehen bzw. die metallische Beschichtung perforiert wird. Am Porengrund wird teilweise auch das Implantatkörper-Material bzw. die metallischen Beschichtung, bis in eine bestimmte, von der konkreten Energie der Mischspannung abhängigen Tiefe ionisiert. Hierdurch entsteht ein enger Stoffschluss zwischen dem Implantatkörper und der plasmachemisch erzeugten Schicht, welcher zu einer hohen Adhäsionsfestigkeit der auf der Implantatkörper-Oberfläche angeordneten Schicht führt. Die plasmachemisch erzeugte Schicht mit den Bestandteilen der metallischen Beschichtung wird abschnittsweise gewissermaßen in die Oberfläche des Implantatkörpers einlegiert. Hieraus folgt, dass die plasmachemisch erzeugte Schicht auch bei hoher mechanischer Beanspruchung des erfindungsgemäßen Implantats, beispielsweise bei der Stentdilatation, nicht von dem Implantatkörper delaminiert. Die Degradation erfolgt daher kontrollierbarer und gleichmäßiger, da lediglich im Bereich der Poren ein definierter Zugang des Elektrolyten zum Material des Implantatkörpers vorhanden ist. Hierbei ist es von Vorteil, wenn die Verteilung der Poren gleichmäßig ist. Die plasmachemisch erzeugte Schicht stellt dort, wo keine Pore angeordnet ist, bei Kontakt mit der Körperflüssigkeit einen temporären Korrosionsschutz dar, der eine verzögerte Degradation bewirkt.

Die Energie ist ein Produkt aus der Badspannung U und dem Strom I bzw. ein Produkt aus Stromdichte und Pulslänge. Vorzugsweise werden zur plasmachemischen Behandlung Energien im Bereich von 0,6 Ws bis 8 Ws verwendet, besonders bevorzugt für Implantate vorzugsweise Stents, enthaltend eine Eisenlegierung, ist der Bereich zwischen 1 Ws und 3 Ws Energie. Die angegebenen Energiewerte gelten insbesondere für Implantate mit einer Gesamtoberfläche in der Größenordnung von ca. 100 mm².

Beispielsweise ergibt sich bei einer mittleren Badspannung von 400 V (Maximalwert der Peakspannung beträgt hierbei 500 V), einer Stromdichte von 1 A/dm² = 10 mA/cm², einer Pulsdauer von 5 µs und 1000 µs Pulspause (ergibt einen Faktor von 0,005) und einer Beschichtungszeit von 1 Minute folgende energetische Beziehung:
Implantatoberfläche eines Stents ca. 100 mm² = 0,01 dm²
Beschichtungsenergie W = U_{eff} · I · t_{eff}.
= 400V · 0,01 dm² · 1 A/dm² · 60 s · 0,005 (pulse on/off)
= 1,2 Ws

Die Oberfläche eines Implantates, wie z. B. eines Stents mit einer Oberfläche von ca. 100 mm², wird daher mit einer Beschichtungsenergie von mindestens ca. 0,6 Wattsekunden plasmachemisch oberflächenbehandelt.

Weiter von Vorteil ist, dass die verfahrensbedingt poröse Struktur der plasmachemisch erzeugten Schicht ein hohes plastisches Verformungsvermögen aufweist. Beispielsweise werden die beim Dilatieren eines Stents entstehenden Mikrorisse durch Energieakkumulation beziehungsweise -dissipation in den zu den Mikrorissen benachbarten Poren gestoppt. Es kommt somit nicht zu einer Delamination der plasmachemisch erzeugten Schicht.

Falls gewünscht, kann noch eine weitere, z. B. polymere Beschichtung auf die plasmachemisch erzeugte Schicht aufgebracht werden. Aufgrund der Porenstruktur ergibt sich eine gute Haftung der weiteren Beschichtung auf der plasmachemisch erzeugten Schicht.

Außerdem kann die Porenstruktur der plasmachemisch erzeugten Schicht als Stoffreservoir für pharmazeutisch aktive Substanzen fungieren, welche als Nano- oder Mikropartikel eingelagert werden können und als Gleitmittel zur Verringerung des Reibungskoeffizienten im Katheter, knochenwachstumsfördernde Substanzen wie Kalziumphosphate, temporär wirkende Kontrastmittel oder zellwachstumshemmende radioaktive Substanzen dienen können.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Umgebung des Implantats hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können beispielsweise aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel, oder Sirolimus, bestehen.

Auf Grund des anodischen Wirkprinzips der plasmachemischen Beschichtung besteht zudem keine Gefahr der Wasserstoffversprödung des Implantatkörper-Materials. Auch die Gefahr einer mechanischen Beschädigung des Implantatkörper-Materials besteht nicht, da das Aufrauen der Oberfläche nicht mechanisch erfolgt.

Ein weiterer Vorteil des erfindungsgemäßen Verfahrens besteht darin, dass nicht entfernbare Oberflächenkontaminationen des Grundmaterials durch die plasmachemisch erzeugte Schicht absorbiert werden und somit den Degradationsprozess nicht zusätzlich beeinflussen. Zudem werden aus der Oberfläche herausragende, teilweise scharfkantige Ausscheidungen des Implantat-Körpers (wie zum Beispiel nicht gelöste Legierungsbestandteile) abgedeckt. Daraus folgt auch eine erhöhte Hämo- bzw. Biokompatibilität.

Aufgrund der Existenz der mittels des erfindungsgemäßen Verfahrens hergestellten plasmachemisch erzeugten Schicht vereinfachen sich zudem die Lager- und Transportbedingungen für nach dem erfindungsgemäßen Verfahren hergestellten Implantate, da die Stabilität unter Lagerbedingungen und im montierten und sterilisierten Zustand eines solchen Implantats gegenüber Degradation höher ist als bei unbeschichteten Implantaten.

In einem bevorzugten Ausführungsbeispiel beträgt die Schichtdicke der metallischen Beschichtung nach Abschluss von Schritt b) 0,1 µm bis 1,5 µm. Die genannte Dicke der metallischen Beschichtung ist derart gewählt, dass die metallische Beschichtung abschnittsweise einschließlich des darunter liegenden Materials des Implantatkörpers mittels realisierbarer plasmachemischer Oxidationseffekte kurzzeitig ionisiert werden kann. Außerdem kann die Degradationsgeschwindigkeit durch Variation der Schichtdicke gesteuert werden.

Hierdurch eröffnet sich auch die Möglichkeit, die Degradationsdauer des Implantats an den spezifischen Implantationsort anzupassen (koronar, intercranial, renal etc.)

Es ist weiterhin von Vorteil, wenn die metallische Beschichtung mittels einer ionischen Flüssigkeit aufgebracht wird. Dies ist ein besonders einfaches Verfahren, um alle Teile der Oberfläche des Implantatkörpers, auch von Hinterschneidungen, gleichmäßig zu beschichten.

Alternativ können auch PVD- und CVD-Verfahren (wie z. B. Sputtern oder thermisches Aufdampfen) zum Aufbringen der metallischen Beschichtung verwendet werden. Allerdings dürfen diese beim Einsatz von Eisen als Material des Implantatkörpers eine Temperatur von ca. 400° C bei einer Behandlungszeit von 30 min nicht überschreiten. Andernfalls würden sich irreversible Änderungen der mechanischen Werkstoffeigenschaften ergeben (Entfestigung). Die Beschichtung mittels PVD- oder CVD-Verfahren haben jedoch gegenüber einer Beschichtung mittels ionischer Flüssigkeit den Nachteil, dass keine einheitliche Bedeckung der häufig kompliziert geformten Implantatoberflächen stattfindet. Deshalb sind PVD- oder CVD-Verfahren insbesondere für Implantate geeignet, die nur einfach geformte Strukturen bzw. Designs aufweisen.

Demgegenüber kann die Beschichtung mittels ionischer Flüssigkeiten für komplizierter geformte Implantate mit Hinterschneidungen, Hohlräumen und/oder Sacklöchern angewendet werden. Ein solches Beschichtungsverfahren erlaubt die Realisierung einer einheitlichen Schichtdicke auch bei komplizierten Formen (z. B. im Inneren von Stents) und trägt damit maßgeblich zu einem uniformen Degradationsverhalten nach der plasmachemischen Behandlung bei.

In einer Weiterbildung der Erfindung weist die metallische Beschichtung neben dem mindestens einen Ventilmetall zusätzlich ein Element der Gruppe enthaltend Kupfer und Silber auf. Der Gesamtanteil der Elemente Kupfer und/oder Silber an dem Material der metallischen Beschichtung soll hierbei vorzugsweise nicht mehr als 15 Masse%, vorzugsweise nicht mehr als 11 Masse% betragen. Insbesondere ist der Silber- und/oder Kupfergehalt so gewählt, dass das Verhältnis des Gehalts Ventilmetall zu Silber oder Kupfer außerhalb der Löslichkeitsgrenze des jeweiligen binären Systems liegt. Durch die plasmachemische Behandlung entsteht in der Matrix des Ventilmetalls gelöstes Silber oder Kupfer bzw. metallisches Kupfer und/oder Silber auf der Oberfläche der plasmachemisch erzeugten Schicht. Der metallische Anteil wirkt im späteren Kontakt mit den Zellen des Körpers des Behandelten antiproliferativ. Hierbei beträgt der Anteil des metallischen Silber und/oder Kupfer an dem Material der plasmachemisch erzeugten Schicht weniger als 5 Masse%.

In einem weiteren Ausführungsbeispiel wird das Implantat nach der plasmachemischen Beschichtung in einem Lösungsmittel, vorzugsweise destilliertem H₂O, gespült und danach vorzugsweise bei einer Temperatur von mindestens etwa 80°C, besonders bevorzugt mindestens etwa 100°C, getrocknet, wobei die Trocknung vorzugsweise in einem Umluftofen durchgeführt wird. Diese Vorgehensweise bewirkt das rückstandslose Entfernen von Verbindungen des plasmachemischen Elektrolyten von der Implantatoberfläche. Die Trocknung bei erhöhten Temperaturen stellt auch sicher, dass eventuell durch Kapillarwirkung länger anhaftende Elektrolytreste (z. B. in der filigranen Oberflächenform eines Stents) keine unerwünschten Reaktionen mit der Implantatoberfläche eingehen.

In einem bevorzugten Ausführungsbeispiel enthält die bei der plasmachemischen Behandlung verwendete wässrige Lösung Sr²⁻ Ionen, die vorzugsweise in einer Konzentration von jeweils 0,05 Mol/l bis 2,0 Mol/l Sr²⁻ in der wässrigen Lösung enthalten sind. Hierdurch werden Strontiumverbindungen in der plasmachemisch erzeugten Schicht gebildet. Diese sind vorteilhaft, da insbesondere Strontiumcarbonat kaum wasserlöslich ist und somit einen die Degradation besonders hemmenden Schichtbestandteil in der Oberflächenschicht des Implantats bildet. Zudem kann das in der Beschichtung enthaltene Strontiumcarbonat insbesondere bei cranialen Anwendungen eine arzneimittelähnliche Wirkung gegen Zerebralsklerose entfalten.

In einem bevorzugten Ausführungsbeispiel des erfindungsgemäßen Verfahrens sind in der wässrigen Lösung, die für die plasmachemische Behandlung verwendet wird, zusätzlich ein oder mehrere Ionen ausgewählt aus der Gruppe der Phosphate, Carbonate, Hydroxide und Silikate enthalten.

Enthält die wässrige Lösung zur plasmachemischen Behandlung Phosphationen, so bilden sich neben den Oxiden und Hydroxiden in der plasmachemisch erzeugten Schicht auch Phosphate des Materials der metallischen Beschichtung oder des Implantatkörpers aus, die für eine bessere biologische Verträglichkeit des Implantatmaterials, insbesondere der Beschichtung, sorgen. Dabei entstammen die Phosphationen der Zugabe von Kaliumdihydrogenphosphat und/oder Dikaliumhydrogenphosphat und/oder Kaliumphosphat und/oder Natriumdihydrogenphosphat (Dihydrat) und/oder Heptahydrat und/oder Dodecahydrat in den wässrigen Elektrolyten. Der bevorzugte Konzentrationsbereich liegt dabei zwischen 5 g/l und 200 g/l der zugegebenen Verbindung in der wässrigen Lösung. Eine besonders bevorzugte Konzentration beträgt zwischen 50 und 100 g/l Kaliumdihydrogenphosphat.

Um den pH-Wert des Elektrolyten (wässrigen Lösung) konstant zu halten, ist bevorzugt in der wässrigen Lösung ein Puffer, vorzugsweise Kaliumdihydrogenphosphat und/oder Natriumdihydrogenphosphat enthalten.

In einem weiteren bevorzugten Ausführungsbeispiel wird der Implantat-Körper vor dem Auftragen der metallischen Beschichtung oder vor der plasmachemischen Behandlung elektrochemisch behandelt, vorzugsweise elektrochemisch poliert. Hierdurch werden Verunreinigungen auf der Oberfläche des Implantat-Körpers entfernt, so dass das Aufbringen der metallischen Beschichtung oder die plasmachemische Behandlung an einer definierten Oberfläche erfolgt. Wichtig ist die Freiheit von Oberflächenkontaminationen, die sonst zu einer schlechten Haftung der plasmachemisch erzeugten Schicht führen würde. Das Elektropolieren führt zu kontaminationsarmen Deckschichten auf Grund großer Materialabtragseffekte (Tiefenwirkung).

Bevorzugt erfolgt die plasmachemische Behandlung des Implantat-Körpers dadurch, dass an den Körper eine gepulste, vorzugsweise positive, Spannung angelegt wird, deren Amplitude (Peakspannung) im überwiegenden Zeitraum der Behandlung mindestens etwa 400 Volt übersteigt, besonders bevorzugt mindestens etwa 450 Volt übersteigt, und welche vorzugsweise im Verlauf der Behandlung bis maximal etwa 500 V ansteigt. Der Spannungsverlauf entspricht des oben mit Misch- oder Impulsspannung bezeichneten Verlaufs.

Durch diese hohen gepulsten Spannungen mit einer Pulslänge von vorzugsweise maximal etwa 50 Mikrosekunden, besonders bevorzugt etwa 5 Mikrosekunden, werden an der Oberfläche des Implantat-Körpers über Mikrosekunden Plasmen erzeugt, welche zur Reaktion des metallischen Materials des Implantatkörpers mit dem Elektrolyten führen. Zwischen den Spannungspulsen folgt eine Ruhephase von vorzugsweise ca. 1000 Mikrosekunden. Die aufzubringende Energie in Form der Spannungspulse beträgt bei einer Implantatoberfläche von 100 mm² mindestens ca. 0,6 Ws. Bei Implantatoberflächen mit einer abweichenden Größe verändert sich die Mindestenergie proportional zum Flächeninhalt der Oberfläche.

Bevorzugt wird der plasmachemische Prozess mit einer Stromdichte von mindestens etwa 5 mA/cm², vorzugsweise mindestens etwa 10 mA/cm² durchgeführt.

In einem weiteren bevorzugten Ausführungsbeispiel wird nach der plasmachemischen Behandlung auf die plasmachemisch erzeugte Schicht eine weitere, vorzugsweise eine Polymer enthaltende Schicht (im Folgenden vierte Schicht genannt), besonders bevorzugt enthaltend mindestens ein Polymer der Gruppe Parylene, PLA (Polylactid) und deren Copolymere, insbesondere PLLA (Poly-L-Lactid) und PLGA (Poly(lactic-co-glycolic acid)), aufgebracht. Bevorzugte Schichtdicken der weiteren Schicht liegen zwischen etwa 0,5 und etwa 10 µm. Durch eine derartige Schichtkombination kann die Degradationszeit des Implantats nochmals wesentlich erhöht werden. Vorteilhaft wirkt sich auch die hohe Spaltgängigkeit von Parylene aus, so dass auch eine tiefgehende Penetration der Poren der plasmachemisch erzeugten Schicht und/oder die Porengrundschicht bis hin zu den Porengründen durch Parylene erfolgt. Die für PLA und Parylene, insbesondere Parylene N, charakteristischen Permeationseigenschaften für Wasser, chloridhaltige Lösungen und Wasserstoff sorgen in Verbindung mit der darunter liegenden plasmachemisch erzeugten Oberfläche für ein steuerbares Degradationsverhalten des Implantats. Dieses zeichnet sich auch durch einen über den Implantatquerschnitt uniformen langsamen Korrosionsfortschritt aus. Zudem leistet die weitere Schicht einen zusätzlichen Beitrag zur Vermeidung bzw. Behinderung des Rissfortschritts bei mechanischer Belastung und verhindert partielle Schichtablösungen. Insbesondere bevorzugt ist, wenn die weitere Schicht aus Parylene N oder PLLA L210 besteht.

Hierbei ist Parylene die Bezeichnung für vollständig lineare, teilkristalline, unvernetzt aromatische Polymere. Die verschiedenen Polymere besitzen unterschiedliche Eigenschaften und lassen sich in vier Grundtypen einteilen, nämlich Parylene C, Parylene D, Parylene N und Parylene F. Für die Anwendung als weitere Schicht im erfindungsgemäßen Schichtverbund wird vorzugsweise Parylene N verwendet.

Die unter der weiteren Schicht angeordnete plasmachemisch erzeugte Schicht führt auf Grund ihrer porösen Struktur zu einem hohen Haftungsvermögen der Polymerschicht, so dass eine sonst vorzuschaltende Primerbehandlung überflüssig wird.

Die obige Aufgabenstellung wird ferner gelöst durch ein Implantat erhältlich oder erhalten durch ein oben beschriebenes erfindungsgemäßes Verfahren. Ein derartiges Implantat weist die oben im Zusammenhang mit dem erfindungsgemäßen Herstellungsverfahren angegebenen Vorteile auf.

Erfindungsgemäß weist die metallische Beschichtung nach Abschluss des Schritts b) eine Schichtdicke von 0,1 µm bis 1 µm auf.

Oben wurde bereits beschrieben, dass die plasmachemisch erzeugte Schicht Poren aufweist, wobei die Poren vorzugsweise eine Dichte von etwa 50.000 Poren/mm² bis etwa 250.000 Poren/mm² haben. Es wurde erkannt, dass in diesem Porendichtebereich noch eine gute Benetzung des Porengrundes mit Blut bzw. anderen Körperflüssigkeiten (Plasma) stattfindet, während bei größeren Porendichten die Benetzungswirkung so klein wäre, dass die Korrosion nicht beschleunigt ablaufen würde. Geringere Porendichten unter 50.000 Poren/mm² würden eine zu hohe Kerbwirkung entfalten. Das bedeutet, dass die Poren bei der Dilatation von Stents die Risse nicht stoppen könnten und dass deshalb die Beschichtung großflächig delaminieren würde.

In einer Weiterbildung der Erfindung weisen die Poren der plasmachemisch erzeugten Schicht einen Durchmesser von etwa 0,5 µm bis etwa 5 µm, vorzugsweise von etwa 1 µm bis etwa 2 µm, auf. Analog zu den obigen Ausführungen zur Porendichte sind große Poren bei gegebener Fläche gleichzusetzen mit kleiner Porendichte. Demzufolge würden größere Poren als die als vorstehend angegebenen zu einer erhöhten Kerbwirkung führen. Bevorzugt enthält die plasmachemisch erzeugte Schicht mindestens eine Verbindung ausgewählt aus der Gruppe enthaltend Phosphate, Hydroxide und Oxide des Materials des Implantatkörpers und des Materials der metallischen Schicht, insbesondere Phosphate, Hydroxide und Oxide des Eisens, des Tantals, des Niobs, des Zirkons, des Aluminiums, des Magnesiums, des Vanadiums, des Molybdäns, des Hafniums und/oder des Wolframs. Hierbei wird ein Zusammensetzungsgradient von der Oberfläche des Implantats in die Tiefe bis zum Übergang der plasmachemischen erzeugten Schicht in das Material des Implantatkörpers beobachtet.

Der Vorteil von Tantal oder einem anderen Ventilmetall als Bestandteil der metallischen Beschichtung und somit auch als Bestandteil der plasmachemischen erzeugten Schicht besteht darin, dass diese Materialien mit dem am Porengrund angeordneten Eisen ein Lokalelement ausbilden, wobei der aus der Lage des Eisens in der elektrochemischen Spannungsreihe resultierende Potentialunterschied zu dem Ventilmetall (verglichen mit Edelmetallen) vergleichsweise gering ist, so dass der Degradationsprozess nicht zu schnell und zu unkontrolliert verläuft.

Ein weiterer Vorteil des erfindungsgemäßen Implantats besteht darin, dass das Umformvermögen des Implantats, z. B. bei der Dilatation, verbessert ist. Dies liegt daran, dass ein Gradient des E-Moduls von der äußeren Oberfläche des erfindungsgemäßen Implantats, also von der äußeren Oberfläche der plasmachemisch erzeugten Schicht, nach innen vorliegt. Dadurch werden die mechanischen Spannungen zwischen der Implantatoberfläche und der neutralen Phase im Inneren des Bauteils wesentlich verringert.

Die erfindungsgemäßen Verfahren werden nachfolgend anhand von Beispielen und Figuren erläutert. Dabei bilden alle abgebildeten und/oder beschriebenen Merkmale den Gegenstand der Erfindung, unabhängig von ihrer Zusammenfassung in den Ansprüchen oder deren Rückbeziehung.

Es zeigen schematisch:
- Figur 1: einen Querschnitt des oberflächennahen Bereichs eines Steges eines ersten Ausführungsbeispiels eines erfindungsgemäßen Stents,
- Figur 2: einen vergrößerten Ausschnitt aus dem in Figur 1 gezeigten Querschnitt und
- Figur 3: einen Querschnitt des oberflächennahen Bereichs eines Steges eines zweiten Ausführungsbeispiels eines erfindungsgemäßen Stents.

### Beispiel 1:

Ein Stent-Körper aus einer Eisenbasis-Legierung (99,5 Masse% Eisen, Rest Kohlenstoff) wird mittels herkömmlicher Verfahren wie Laserschneiden und Elektropolieren endkonturnah gefertigt. Anschließend wird der Stent-Körper mittels einer ionischen Flüssigkeit mit einer metallischen Beschichtung bestehend aus Tantal und 10 Masse% Silber versehen, wobei die Schichtdicke der metallischen Beschichtung etwa 1 µm beträgt.

Die Beschichtung des Stent-Körpers mittels einer ionischen Flüssigkeit wird folgendermaßen realisiert. Der Elektrolyt ist eine ionische Flüssigkeit und setzt sich zusammen aus 1-Butyl-1-methylpyridinium, Trifluoromethylsulfamat, TaF₅ und Silbertrifluoromethylsulfamat. Die Beschichtung erfolgt bei einer Temperatur der ionischen Flüssigkeit von 100 - 250°C. Die Beschichtungszeit beträgt etwa 0,1 Stunden. Die potentiostatische Polarisierung beträgt ca. -100 mV bis -1000 mV versus. Ta/TaF₅.

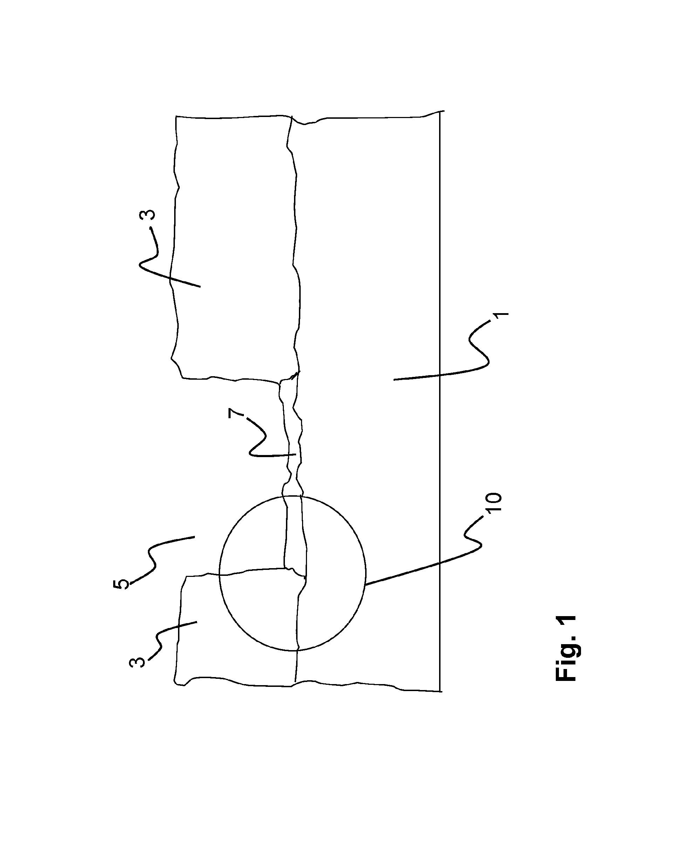

Anschließend wird eine plasmachemische Oxidation des mit der metallischen Beschichtung versehenen Stents in einem wässrigen, phosphathaltigen Elektrolyten durchgeführt. Hierdurch entsteht eine ca. 1 µm dicke plasmachemisch erzeugte Schicht mit regelmäßig angeordneten Poren von ca. 1 µm bis 2 µm Durchmesser und einer Porendichte von 50.000 bis 250.000 Poren/mm². Ein Querschnitt einer Strebe eines so hergestellten Stents ist in Figur 1 dargestellt.

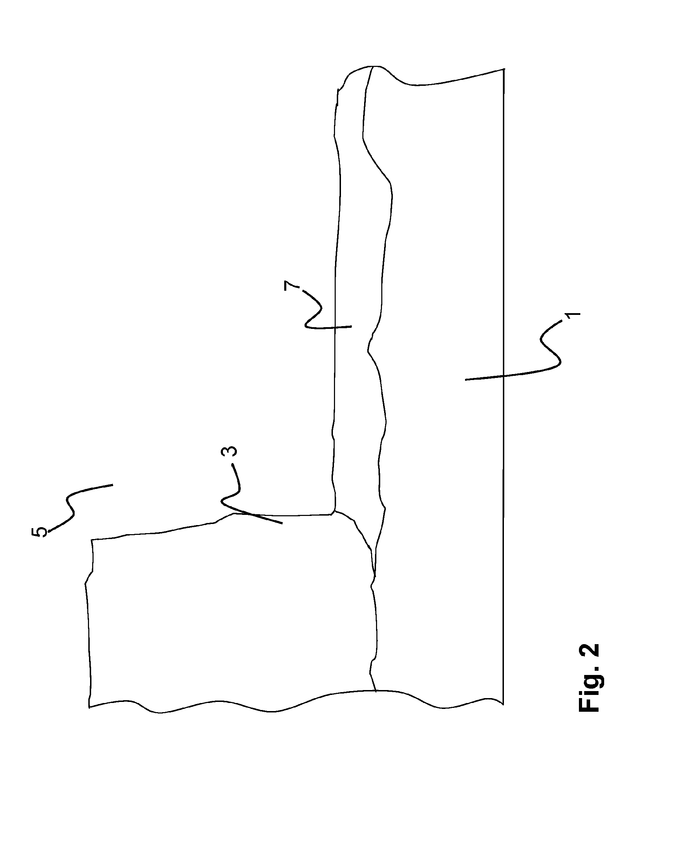

Auf dem Implantatkörper 1 aus der Eisenbasislegierung ist die plasmachemisch erzeugte Schicht 3 angeordnet, welche Poren 5 aufweist. Die Schichtdicke der plasmachemisch erzeugten Schicht 3 beträgt etwa 1 µm. Die Pore 5 hat einen Durchmesser von ca. 1,5 µm. Der eingekreiste Ausschnitt 10 des in Figur 1 gezeigten Querschnitts ist in Figur 2 noch einmal vergrößert dargestellt.

In dem vergrößerten Ausschnitt 10 ist zu erkennen, dass der Grund der Pore 5 durch eine etwa 200 nm dicke, auf dem Basiswerkstoff (Eisenbasis-Legierung) des Implantatkörpers 1 angeordnete Schicht 7 gebildet wird, welche aus Mischoxiden und Mischphosphaten des Materials der metallischen Beschichtung (Tantal und Silber) und des Grundwerkstoffs (Eisenbasis-Legierung) besteht. Demgegenüber wird das seitlich zu der Pore 5 angeordnete Material der plasmachemisch erzeugten Schicht 3 lediglich aus Oxiden und Phosphaten des Materials der metallischen Beschichtung (Tantal und Silber) gebildet. Es soll an dieser Stelle nochmals betont werden, dass die Porengrundschicht 7 und die Poren 5 Bestandteile der plasmachemisch erzeugten Schicht 3 sind.

Das Verfahren der Herstellung der plasmachemisch erzeugten Schicht beinhaltet beispielsweise die folgenden Schritte:
- Kontaktieren des Stent-Körpers mit einem Draht aus Titan.
- Eintauchen des mit der Beschichtung versehenen Stent-Körpers in den wässrigen Elektrolyt mit der folgenden Zusammensetzung (wässrige Basis): 20 g/l Ammoniumcarbonat ((NH₄)₂CO₃ · H₂O)
   20 g/l Kaliumdihydrogenphosphat (KH₂PO₄
   20 g/l Natriumcarbonat (Na₂CO₃)
   100 ml/l wässrige Ammoniaklösung (25%)
   100 ml/l Ethylendiamin (C₂H₈N₂).
- Beaufschlagung mit einer stetig steigenden gepulsten Gleichspannung von 0 V aufwärts, wobei der Verlauf der Spannung dem oben als Misch- oder Impulsspannung bezeichneten Verlauf entspricht und der Stent-Körper als Anode geschaltet ist.
- Die Endspannung (Maximalwert der Peakspannung) von 500 V wird nach ca. 1 Minute erreicht.
- Die mittlere Spannung beträgt ca. 400 V, da der untere Badspannungsbereich sehr schnell durchlaufen wird.
- Herausnehmen des nun mit der plasmachemisch erzeugten Schicht versehenen StentKörpers aus dem Elektrolyt und Entkontaktieren an Luft und
- Trocknen des Stents wie oben beschrieben.

Durch den wässrigen phosphathaltigen Elektrolyten werden die Oberfläche des Implantatkörpers und die metallische Beschichtung abschnittsweise in ihrer chemischen Zusammensetzung verändert. Es entstehen in verschiedenen Tiefen zur Oberfläche der metallischen Beschichtung verschiedene Tantal- und Eisen-Mischverbindungen sowie teilweise in der metallischen Matrix gelöstes Silber sowie metallisches Silber an der Oberfläche der plasmachemisch erzeugten Schicht. Der an der Oberfläche angeordnete Silber-Anteil wurde durch die plasmachemische Behandlung nicht umgewandelt. Dieses metallische Silber mit einem Anteil an der plasmachemisch erzeugten Schicht von < 5 Masse% wirkt bei einem Kontakt mit Körperzellen während der Behandlung eines Menschen oder eines Tiers antiproliferativ.

Der so behandelte Stent wird anschließend gecrimpt, montiert, zur Behandlung eines Menschen oder Tiers mittels eines Katheters eingeführt und am z. B. koronaren Bestimmungsort dilatiert. Durch die hohe Adhäsion des plasmachemisch modifizierten Tantal zum Eisen-Bulkmaterial erfolgt während des Dilatationsprozesses keine Schichtdelamination und die Korrosion kann unabhängig von der Stentgeometrie und der jeweils vorliegenden Zone plastischer Verformungen einsetzen. Die überall auf der Stentoberfläche vorhandene poröse Struktur der plasmachemisch erzeugten Schicht, welche mit dem Vorhandensein von metallischem bzw. hydroxidischem Eisen am Porengrund einhergeht, wird nun korrodiert, wobei das restliche Tantal als Lokalelement und Eisen als Opferanode wirkt. Bedingt durch die hohe arteigene Korrosionsbeständigkeit des Tantals kann dieses trotz seiner im Vergleich zum Eisen geringen Masse über die gesamte Degradationszeit als Lokalelement wirken. Nach einer Degradationszeit von ca. 12 Monaten wurde der nach Beispiel 1 hergestellte Stent verstoffwechselt (d. h. es verliert nach ca. 12 Monaten seine Integrität) und etwaige noch vorhandene Partikel aus metallischem Tantal bzw. andere Tantal-Verbindungen wurden ohne negative Begleiterscheinungen in die Gefäßwand inkorporiert. Zu Beginn der Degradation verhinderten die sich in dieser Umgebung bildenden Silberionen wirksam die Bildung von Neointima und trugen somit wesentlich zu einem stabilen Lumen über die Standzeit des Stents bei.

### Beispiel 2:

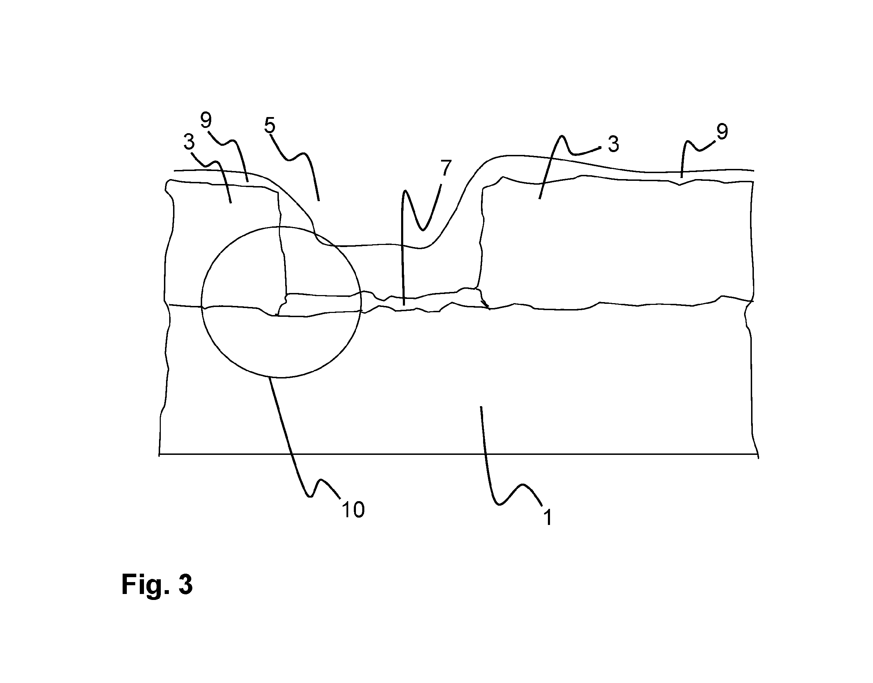

Das Beispiel 2 entspricht dem Beispiel 1 mit dem Unterschied, dass die metallische Beschichtung lediglich mit Tantal (ohne Silber) erfolgt. Ferner der Stent wird nach der plasmachemischen Behandlung zusätzlich mit einem degradierbaren, ggf. sauer abbaubaren Polymer, beispielsweise mit PLGA 85/15 oder PLLA L210, mittels bekannter Verfahren besprüht. Hierbei ist in das Polymer eine pharmazeutisch aktive Substanz, beispielsweise Paclitaxel oder Sirolimus, eingelagert. Ein Querschnitt einer Strebe mit einer Polymer-Beschichtung ist in Figur 3 dargestellt. Die Polymer-Schicht ist mit dem Bezugszeichen 9 versehen und hat eine Schichtdicke von etwa 1 bis 3 µm. Die übrigen, in Figur 3 gezeigten Schichten entsprechen der in Figur 1 gezeigten Anordnung.

Der so behandelte Stent wird anschließend gecrimpt, montiert, zur Behandlung eines Menschen oder Tiers mittels eines Katheters eingeführt und am z. B. koronaren Bestimmungsort dilatiert. Durch die hohe Adhäsion des plasmachemisch modifizierten Tantal zum Eisen-Bulkmaterial des Stentkörpers erfolgt während des Dilatationsprozesses keine Schichtdelamination und die Korrosion kann unabhängig von der Stentgeometrie und der jeweils vorliegenden Zone plastischer Verformungen einsetzen. Die überall auf der Stentoberfläche vorhandene poröse Struktur der plasmachemisch erzeugten Schicht, welche mit dem Vorhandensein von Verbindungen des Eisens (oxidisch und/ oder hydroxidisch und/ oder phosphatisch am Porengrund einhergeht, wird nun korrodiert, wobei das restliche Tantal als Lokalelement und Eisen als Opferanode wirkt.

Die Polymerschicht wirkt zu Beginn des Degradationsprozesses als Diffusionsbarriere für das korrosive Medium. Das heißt, es kommt zunächst zu einer Verlangsamung des Degradationsprozesses. Mit zunehmendem Abbau des Polymers wird ein Korrosionsprozess initiiert, der dem des Beispiels 1 nahe kommt. Die nun vorhandenen sauren Abbauprodukte des Polymers beschleunigen jedoch den Degradationsprozess. Der Vorteil dieses Beispiels besteht also darin, dass die Stützwirkung des Stents am Anfang länger erhalten bleibt, mit weitergehender Degradation aber schneller nachlässt als im Beispiel 1 beschrieben. Trotzdem verbleibt eine längere integrale Degradationszeit von ca. 15 Monaten. Etwaige, noch vorhandene Partikel aus metallischem Tantal bzw. andere Tantal-Verbindungen werden ohne negative Begleiterscheinungen in die Gefäßwand inkorporiert. Referenzstents aus unbehandeltem Eisen weisen eine Degradationszeit von 24 Monaten auf.

### Bezugszeichenliste

- 1: Implantatgrundkörper
- 3: plasmachemisch erzeugte Schicht
- 5: Pore
- 7: Porengrundschicht
- 9: weitere Schicht
- 10: Ausschnitt

## Patentansprüche

1. Verfahren zur Herstellung eines Implantats, insbesondere einer intraluminalen Endoprothese, mit einem Körper, wobei der Körper Eisen oder eine Eisenlegierung enthält, umfassend die folgenden Schritte:
a) Bereitstellen des Implantatkörpers (1),
b) Aufbringen einer metallischen Beschichtung mit dem Hauptbestandteil mindestens eines Elements der Gruppe enthaltend Tantal, Niob, Zirkon, Aluminium, Magnesium, Vanadium, Molybdän, Hafnium und Wolfram auf mindestens einen Teil der Körperoberfläche,
c) plasmachemische Behandlung des mit der Beschichtung versehenen Teils der Körperoberfläche in einer wässrigen Lösung zur Herstellung einer plasmachemisch erzeugten Schicht (3, 5, 7) mittels Anlegen einer das Plasma generierenden Mischspannung an den Körper (1) des Implantats, wobei die Mischspannung eine ausreichende Energie dafür aufweist, dass die metallische Beschichtung (3, 5, 7) zeitweise und abschnittsweise derart bis zu dem darunter liegenden Implantatkörper ionisiert wird, dass die plasmachemisch erzeugte Schicht zumindest teilweise bis zum Implantatkörper (3, 5, 7) durchgehende Poren (5) aufweist.

2. Verfahren nach Anspruch 1, **dadurch gekennzeichnet, dass** die Schichtdicke der metallischen Beschichtung nach Abschluss von Schritt b) etwa 0,1 µm bis etwa 1,5 µm beträgt.

3. Verfahren nach einem der vorhergehenden Ansprüche **dadurch gekennzeichnet, dass** die metallische Beschichtung mittels einer ionischen Flüssigkeit, mittels eines PVD-Verfahrens oder mittels eines CVD-Verfahrens aufgebracht wird.

4. Verfahren nach einem der vorhergehenden Ansprüche **dadurch gekennzeichnet, dass** die metallische Beschichtung zusätzlich ein Element der Gruppe enthaltend Kupfer und Silber aufweist.

5. Verfahren nach einem der vorhergehenden Ansprüche, **dadurch gekennzeichnet, dass** das Implantat nach Schritt c) in einem Lösungsmittel, vorzugsweise mittels destilliertem H₂O, gespült und anschließend vorzugsweise bei einer Temperatur von mindestens etwa 80°C, besonders bevorzugt mindestens etwa 100°C, getrocknet wird, wobei die Trocknung vorzugsweise in einem Umluftofen durchgeführt wird.

6. Verfahren nach einem der vorhergehenden Ansprüche, **dadurch gekennzeichnet, dass** die wässrige Lösung Sr²⁻-Ionen enthält, die vorzugsweise in einer Konzentration von jeweils 0,05 Mol/l bis 2,0 Mol/l Sr²⁻ in der wässrigen Lösung enthalten sind.

7. Verfahren nach einem der vorhergehenden Ansprüche, **dadurch gekennzeichnet, dass** die wässrige Lösung zusätzlich ein oder mehrere Ionen ausgewählt aus der Gruppe der Phosphate, Carbonate und Silikate aufweist und/oder dass in der wässrigen Lösung ein Puffer, vorzugsweise Kaliumdihydrogenphosphat und/oder Natriumdihydrogenphosphat enthalten ist.

8. Verfahren nach einem der vorhergehenden Ansprüche, **dadurch gekennzeichnet, dass** die plasmachemische Behandlung der Körperoberfläche dadurch erfolgt, dass an den Körper eine gepulste Spannung angelegt wird, deren Peakspannung im überwiegenden Teil des Behandlungszeitraums mindestens etwa 400 V übersteigt, besonders bevorzugt mindestens etwa 450 V übersteigt, und vorzugsweise im Verlauf der Behandlung bis etwa 500 V ansteigt, wobei die Stromdichte bei der plasmachemischen Behandlung mindestens etwa 5 mA/cm², vorzugsweise mindestens etwa 10 mA/cm² beträgt.

9. Verfahren nach einem der vorhergehenden Ansprüche, **dadurch gekennzeichnet, dass** auf der plasmachemisch erzeugten Schicht (3, 5, 7) eine weitere, vorzugsweise ein Polymer enthaltende Schicht (9), besonders bevorzugt enthaltend mindestens ein Polymer der Gruppe Parylene, PLA und deren Copolymere, insbesondere PLLA und/oder PLGA, aufgebracht wird.

10. Implantat, insbesondere intraluminale Endoprothese, mit einem Körper, der Eisen oder eine Eisenlegierung aufweist, erhältlich durch ein Verfahren gemäß einem der vorhergehenden Ansprüche.

11. Implantat nach Anspruch 10, **dadurch gekennzeichnet, dass** die Schichtdicke der metallischen Beschichtung nach Abschluss von Schritt b) etwa 0,1 µm bis etwa 1,5 µm beträgt.

12. Implantat nach einem der Ansprüche 10 bis 11, **dadurch gekennzeichnet, dass** die plasmachemisch erzeugte Schicht (3, 5, 7) Poren (5) mit einer Dichte von etwa 50.000 Poren/mm² bis etwa 250.000 Poren/mm² aufweist.

13. Implantat nach einem der Ansprüche 10 bis 12, **dadurch gekennzeichnet, dass** die plasmachemisch erzeugte Schicht (3, 5, 7) Poren (5) mit einem Durchmesser von etwa 0,5 µm bis etwa 5 µm, vorzugsweise von etwa 1 µm bis etwa 2 µm, aufweist.

14. Implantat nach einem der Ansprüche 10 bis 13, **dadurch gekennzeichnet, dass** die plasmachemisch erzeugte Schicht (3, 5, 7) mindestens eine Verbindung ausgewählt aus der Gruppe enthaltend Phosphate, Hydroxide und Oxide des Materials des Implantatkörpers und des Materials der metallischen Schicht, insbesondere Phosphate, Hydroxide und Oxide des Eisens, des Tantals, des Niobs, des Zirkons, des Aluminiums, des Magnesiums, des Vanadiums, des Molybdäns, des Hafniums und/oder des Wolframs aufweist.
